# EUROPEAN PATENT APPLICATION

(11) **EP 2 289 563 A1**
(43) Date of publication of application: **02.03.2011**
(21) Application number: 10174113.0
(22) Date of filing: 26.08.2010
(51) Int. Cl.: A61K 49/00

(54) **Near infrared fluorescent imaging agent**

(30) Priority: 28.08.2009 JP 2009197726
(71) Applicant: Fujifilm Corporation, Minato-ku Tokyo 106-0031 (JP)
(72) Inventor: Koori, Hiroshi, Kanagawa 258-8577 (JP); Taniguchi, Masahiko, Kanagawa 258-8577 (JP)
(74) Representative: HOFFMANN EITLE

(57) **Abstract**

A compound represented by general formula (I) below, or a pharmacologically acceptable salt thereof: 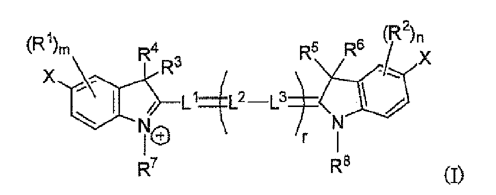 wherein X represents sulfonic acid group or phosphoric acid group; R¹ and R² represent substituents; R³ to R⁶ represent optionally substituted alkyl groups; R⁷ and R⁸ represent optionally substituted alkyl groups; L¹ to L³ represent optionally substituted methine groups; r represents an integer of from 0 to 3, and when r is 2 or more, the plural L² and the plural L³ may be the same or different, respectively; each of m and n represent integers of 0 to 3; at least one of the substituents is an acid group selected from sulfonic acid group, carboxylic acid group, and phosphonic acid group or a group comprising the acid group; and two or more of the acid groups and X are selected from carboxylic acid group and phosphonic acid group.

## Description

### Technical Field

The present invention relates to a near infrared fluorescence imaging agent containing a novel compound.

### Background Art

In the treatment of disease, morphological and functional changes that are caused by disease within an organism are important for detecting disease at an early stage. In the treatment of cancer, in particular, learning the position, size, and nature of a tumor early on is extremely important for determining a strategy and protocol. Examples of methods that have conventionally been employed for this purpose, in addition to biopsy by puncture and the like, are image diagnosis by X-ray imaging, MRI, PET, and ultrasound imaging. Biopsy is useful for making a diagnosis, but the burden on the patient is great and it is unsuitable for tracking the progression of lesions over time. X-ray imaging, MRI, and PET necessarily expose the patient to radiation and electromagnetic waves. Further, in conventional imaging diagnosis such as that mentioned above, complex operations and long periods are required for measurement. From the perspective of the size and shape of the device, it is difficult to perform surgery while navigating using these methods during surgical operations.

Fluorescence imaging is an imaging diagnostic method that solves these problems. In this method, a substance that generates fluorescence when exposed to stimulating light of a specific wavelength is employed as an imaging agent. Further, this method comprises the steps of irradiating an excitation beam from outside the organism and detecting the fluorescence that is released by the fluorescent material within the organism.

Cyanine compounds are typical compounds that can be used in the fluorescence imaging method. In practice, indocyanine green (abbreviated as "ICG" hereinafter) is currently employed to test liver function and in ophthalmic angiography. However, ICG dissolves readily, and must be employed in formulations to which iodine is added. There is thus a problem in that it cannot be used in patients who are allergic to iodine.

The compound (denoted as "SF-64" hereinafter) that is described in Claim 1 of Japanese Patent No. 3,507,060 is an example of a cyanine compound that solves this solubility problem. This dye is characterized by good solubility in water and low toxicity. It is also described in this patent as having the property of outlining tumors.

### Summary of the Invention

The object of the present invention is to provide a novel compound that is useful as an imaging component in near infrared fluorescence imaging agent.

The present inventors conducted extensive research on the mechanisms by which cyanine compounds accumulate in tumors using SF-64. As a result, they discovered that the cyanine compounds and the derivatives of the cyanine compounds form complexes with albumin in plasma. Based on this knowledge, they discovered a new group of cyanine compounds with actually enhanced imaging function relative to SF-64 in terms of the tumor outlining time period. They also discovered that the new group of cyanine compounds exhibit little difference of tumor outlining ability among the animal species. Therefore, the result that will be obtained when the test compound is administered to human can be easily predicted from the result of the animal test, and thus a compound that has high ability to outline a tumor in human can be selected at an early stage of a research. The present invention was devised based on this discovery. The present invention was achieved on the basis of these findings. The present invention thus provides [1] to [11] below.

[1] A compound represented by general formula (I) below, or a pharmacologically acceptable salt thereof: 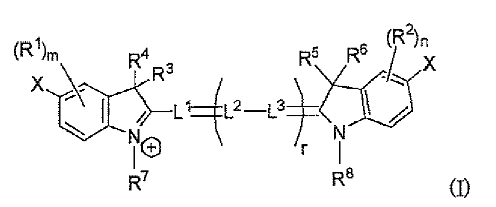 wherein X represents sulfonic acid group or phosphoric acid group; each of R¹ and R² independently represents a substituent; R³ to R⁶ may be identical or different and each represents an optionally substituted alkyl group; each of R⁷ and R⁸ independently represents an optionally substituted alkyl group; L¹ to L⁸ may be identical or different and each represents an optionally substituted methine group; r represents an integer of from 0 to 3, and when r is 2 or more, the plural L² and the plural L³ may be the same or different, respectively; each of m and n independently represents an integer of 0 to 3; at least one of the substituents is an acid group selected from sulfonic acid group, carboxylic acid group, and phosphonic acid group or a group comprising the acid group; and two or more of the acid groups and X are selected form carboxylic acid group and phosphonic acid group.

[2] The compound according to [1] represented by general formula (II) below, or a pharmacologically acceptable salt thereof: 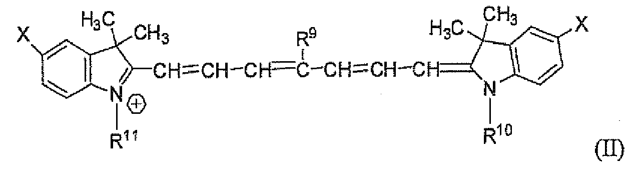 wherein each of R¹⁰ and R¹¹, which may be identical or different, represents an alkyl group substituted with carboxylic acid group, sulfonic acid group, or phosphonic acid group; and R⁹ represents hydrogen atom, an alkyl group, an aryl group, an arylalkyl group, a heterocyclic group, an acylamino group, an arylamino group, an N-aryl-N-alkylamino group, an arylthio group, an aryloxy group, or an acylaminoaryloxy group.

[3] The compound according to [2], or a pharmacologically acceptable salt thereof, wherein R⁹ represents hydrogen atom, methyl group, or phenyl group.

[4] The compound according to [2] or [3], or a pharmacologically acceptable salt thereof, wherein each of R¹⁰ and R¹¹ represents an alkyl group substituted with sulfonic acid group or phosphonic acid group.

[5] The compound according to [2] or [3], or a pharmacologically acceptable salt thereof, wherein each of R¹⁰ and R¹¹ represents an alkyl group substituted with phosphonic acid group.

[6] The compound according to any one of [1] to [5], or a pharmacologically acceptable salt thereof, wherein X represents sulfonic acid group.

[7] The compound according to any one of [1] to [6], or a pharmacologically acceptable salt thereof, wherein one or more of carboxylic acid group, sulfonic acid group, and phosphonic acid group form salts with sodium ions.

[8] A near infrared fluorescence imaging agent comprising the compound, or pharmacologically acceptable salt thereof, according to any one of [1] to [7].

[9] The near infrared fluorescence imaging agent according to [8], which is used to image a tumor.

[10] The near infrared fluorescence imaging agent according to [9], which is used to image a tumor of a type expressing a high level of SPARC.

[11] The near infrared fluorescence imaging agent according to [9] or [10], which is used to image tumors occurring in the mouth cavity, pharynx, digestive organs, respiratory organs, bones, joints, soft tissue, skin, breasts, genitalia, urinary system, eyes, eyeball sockets, brain, central nervous system, or endocrine system.
From additional perspectives, the present invention provides the use of the above compounds for manufacture of a fluorescence imaging agent; a fluorescence imaging method comprising the step of fluorescence imaging after administering the above compound to a mammal, including a human; and a tumor imaging method comprising the step of fluorescence imaging after administering the above compound to a mammal, including a human.

Modes of Carrying Out the Invention

In the present specification, the term "substituent," or the substituent meant by the use of the term "optionally substituted" or "substituted or unsubstituted," can be selected from the group of substituents listed below by way of example:
Substituent group: halogen atoms, alkyl groups, alkenyl groups, alkynyl groups, aryl groups, heterocyclic groups, cyano groups, hydroxyl groups, nitro groups, carboxylic acid groups (carboxyl groups), alkoxy groups, aryloxy groups, silyloxy groups, heterocyclic oxy groups, acyloxy groups, carbamoyloxy groups, alkoxycarbonyloxy groups, aryloxycarbonyloxy groups, amino groups, alkylamino groups, arylamino groups, acylamino groups, aminocarbonylamino groups, alkoxycarbonylamino groups, aryloxycarbonylamino groups, sulfamoylamino groups, alkyl and arylsulfonylamino groups, mercapto groups, alkylthio groups, arylthio groups, heterocyclic thio groups, sulfamoyl groups, sulfonic acid groups (sulfo groups), alkylsulfinyl groups, arylsulfinyl groups, alkylsulfonyl groups, arylsulfonyl groups, acyl groups, aryloxycarbonyl groups, alkoxycarbonyl groups, carbamoyl groups, arylazo groups, heterocyclic azo groups, imide groups, phosphonic acid groups (phosphoric acid groups), phosphino groups, phosphinyl groups, phosphinyloxy groups, phosphinylamino groups, and silyl groups.

Those among these groups that are capable of forming salts, and all that are capable of forming salts by dissociation of one or more hydrogen ions, may be in the form of salts. The counter ions of these salts are, for example, positive charge or negative charge that may be present in the compound of the present invention, an alkali metal ion and an alkaline earth metal ion.

Examples of the halogen atom include chlorine atom, bromine atom, and iodine atom.

Alkyl groups may be linear, branched, cyclic alkyl groups, or any combination thereof. Examples include linear alkyl groups having 1 to 30 carbon atoms (such as methyl, ethyl, n-propyl, isopropyl, t-butyl, n-octyl, eicosyl, and 2-ethylhexyl), cycloalkyl groups having 3 to 30 carbon atoms (such as cyclohexyl, cyclopentyl, 4-n-dodecylcyclohexyl), bicycloalkyl groups having 5 to 30 carbon atoms (monovalent groups obtained by removing a single hydrogen atom from a bicycloalkane having 5 to 30 carbon atoms, such as bicyclo[1,2,2]heptane,2-yl and bicyclo[2,2,2]octane-3-yl, and alkyl groups having structures with even more rings, such as tricyclo structures.

Unless specifically stated otherwise, the alkyl moieties of substituents on alkyl moieties are identical to the above alkyl groups.

Alkenyl groups may be linear, branched, or cyclic alkenyl groups. Examples include linear alkenyl groups having from 2 to 30 carbon atoms (such as vinyl, allyl, prenyl, geranyl, and oleyl), cycloalkenyl groups having 3 to 30 carbon atoms (monovalent groups obtained by removing a single hydrogen atom from a cycloalkene having 3 to 30 carbon atoms, such as 2-cyclopentene-1-yl and 2-cyclohexene-1-yl), and bicycloalkenyl groups having 5 to 30 carbon atoms (monovalent groups obtained by removing a single hydrogen atom from a bicycloalkane having a single double bond, such as bicyclo[2,2,1]hepto-2-ene-1-yl and bicyclo[2,2,2]octo-2-ene-4-yl). Preferable examples of alkynyl groups include alkynyl groups having 2 to 30 carbon atoms (such as ethynyl and propargyl). Preferable examples of aryl groups include aryl groups having 6 to 30 carbon atoms, such as phenyl, p-tolyl, and naphthyl.

Heterocyclic groups are preferably five or six-membered heterocyclic groups, and can be monovalent groups obtained by removing a single hydrogen atom from an aromatic or non-aromatic heterocyclic compound. Preferable examples include five or six-membered aromatic heterocyclic groups having 3 to 30 carbon atoms, such as 2-furyl, 2-thienyl, 2-pyrimidinyl, and 2-benzothiazolyl. Preferable examples of alkoxy groups include alkoxy groups having 1 to 30 carbon atoms, such as methoxy, ethoxy, isopropoxy, t-butoxy, and n-octyloxy. Preferable examples of aryloxy groups include aryloxy groups having 6 to 30 carbon atoms, such as phenoxy groups. Preferable examples of silyloxy groups include silyloxy groups having 3 to 20 carbon atoms, such as trimethylsilyloxy and t-butyldimethylsilyloxy.

Preferable examples of heteroeyclicoxy groups include heterocyclicoxy groups having 2 to 30 carbon atoms, such as tetrazole-5-oxy and 2-tetrahydropyranyloxy. Examples of acyloxyl groups include formyloxy groups, alkylcarbonyloxy groups having 2 to 30 carbon atoms, and arylcarbonyloxy groups having 6 to 30 carbon atoms, such as formyloxy, acetyloxy, pivaloyloxy, stearoyloxy, benzoyloxy, and phenylcarbonyloxy. Preferable examples of carbamoyloxy groups include carbamoyloxy groups having 1 to 30 carbon atoms, such as N,N-dimethylcarbamoyloxy, N,N-diethylcarbamoyloxy, marphalinocarbonyloxy, N,N-di-n-octylaminocarbonyloxy, and N-n-octylcarbamoyloxy. Preferable examples of alkoxycarbonyloxy groups are alkoxycarbonyloxy groups having 2 to 30 carbon atoms, such as methoxycarbonyloxy, ethoxycarbonyloxy, t-butoxycarbonyloxy, and n-octylcarbonyloxy.

Preferable examples of aryloxycarbonyloxy groups include aryloxycarbonyloxy groups having 7 to 30 carbon atoms, such as phenoxycarbonyloxy. Examples of alkylamino groups include alkylamino groups having 1 to 30 carbon atoms, such as methylamino and dimethylamino. Preferable examples of arylamino groups include arylamino groups having 6 to 30 carbon atoms, such as anilino and diphenylamino. Preferable examples of acylamino groups include formylamino groups, alkylcarbonylamino groups having 1 to 30 carbon atoms, and arylcarbonylamino groups having 6 to 30 carbon atoms, such as formylamino, acetylamino, pivaloylamino, lauroylamino, benzoylamino, and 3,4,5-tri-n-octyloxyphenylearbonylamino. Preferable examples of aminocarbonylamino groups include azninacarbanylamino groups having 1 to 30 carbon atoms, such as carbamoylamino, N,N-dimethylaminocarbonylamino, N,N-diethylaminocarbonylamino, and morpholinocarbonylamino.

Preferable examples of alkoxycarbonylamino groups include alkoxycarbonylamino groups having 2 to 30 carbon atoms, such as methoxycarbonylamino, ethoxycarbonylamino, t-butoxycarbonylamino, and n-octadecyloxycarbonylamino. Preferable examples of aryloxycarbonylamino groups include aryloxycarbonylamino groups having 7 to 30 carbon atoms, such as phenoxycarbonylamino. Preferable examples of sulfamoylamino groups include sulfamoylamino groups having 0 to 30 carbon atoms, such as sulfamoylamino, N,N-dimethylaminosulfonylamino, and N-n-octylaminosulfonylamino. Preferable examples of alkyl- and arylsulfonylamino groups include alkylsulfonylamino groups having 1 to 30 carbon atoms and arylsulfonylamino groups having 6 to 30 carbon atoms, such as methylsulfonylamino, butylsulfonylamino, and phenylsulfonylamino. Preferable examples of alkylthio groups include alkylthio groups having 1 to 30 carbon atoms, such as methylthio, ethylthio, and n-lsexadecylthio. Preferable examples of arylthio groups include arylthio groups having 6 to 30 carbon atoms, such as phenylthio. Preferable examples of heterocyclicthio groups are heterocyclicthio groups having 2 to 30 carbon atoms, such as 2-benzothiazolylthio and tetrazole-5-ylthio.

Preferable examples of sulfamoyl groups are substituted or unsubstituted sulfamoyl groups having 0 to 30 carbon atoms, such as N-ethylsulfaxnoyl, N-(3-dodecyloxypropyl)sulfamoyl, N,N-dzznetbylsulfamoyl, N-acetylsulfamoyl, N-benzoylsulfamoyl, and N-(N'-phenylcarbamoyl)sulfamoyl. Preferable examples of alkylsulfinyl groups and arylsulfinyl groups include alkyl sulfinyl groups having 1 to 30 carbon atoms and arylsulfinyl groups having 6 to 30 carbon atoms, such as methylsulfinyl, ethylsulfinyl, and phenylsulfinyl. Preferable examples of alliyl- and arylsulfonyl groups include alkylsulfonyl groups having 1 to 30 carbon atoms, and arylsulfonyl groups having 6 to 30 carbon atoms, such as methylsulfonyl, ethylsulfonyl, and phenylsulfonyl. Preferable examples of acyl groups include formyl groups, alkylcarbonyl groups having 2 to 30 carbon atoms, arylcarbonyl groups having 7 to 30 carbon atoms, and heterocyclic carbonyl groups, having 4 to 30 carbon atoms, that are bonded to carbonyl groups, such as acetyl, pivaloyl, stearoyl, benzoyl, 2-pyridylcarbonyl, and 2-furylcarbonyl. Preferable examples of aryloxycarbonyl groups include aryloxycarbonyl groups having 7 to 30 carbon atoms, such as phenoxycarbonyl.

Preferable examples of alkoxycarbonyl groups include alkoxycarbonyl groups having 2 to 30 carbon atoms, such as methoxycarbonyl, ethoxycarbonyl, t-butoxycarbonyl, and n-octadecyloxycarbonyl. Preferable examples of carbamoyl groups include carbamoyl groups having 1 to 30 carbon atoms, such as carbamoyl, N-methylearbamoyl, N,N-dimethylcarbamoyl, N,N-di-n-octylearbamoyl, and N-(methylsulfonyl)carbamoyl. Preferable examples of arylazo groups and heterocyclic azo groups include arylazo groups having 6 to 30 carbon atoms and heterocyclic azo groups having 3 to 30 carbon atoms, such as phenylazo and 1,3,4-thiadiazole-2-ylazo. Preferable examples of imide groups include N-succinimide and N-phthalimide. Preferable examples of phosphino groups include phosphino groups having 2 to 30 carbon atoms, such as dimethylphosphino, diphenylphosphino, and methylphenoxyphosphino. Preferable examples of phospbinyl groups include phosphinyl groups having 2 to 30 carbon atoms, such as phosphinyl, dioctyloxyphosphinyl, and diethoxyphosphinyl. Preferable examples of phosphinyloxy groups include phosphinyloxy groups having 2 to 30 carbon atoms, such as diphenoxyphosphinyloxy and dioctylaxyphosphinyloxy. Preferable examples of phosphinylamino groups include phosphinylamino groups having 2 to 30 carbon atoms, such as dimethoxyphosphinylamino and dimethylaminophospbinylamino. Preferable examples of silyl groups include silyl groups having 3 to 30 carbon atoms, such as trimethylsilyl, t-butyldimethylsilyl, and phenyldimethylsilyl.

The above substituents may themselves be substituted with the above substituents. Examples of such substituents include arylalkyl groups, acylaminoaryloxy groups, alkylcarbonylaminosulfonyl groups, arylcarbonylaminosulfonyl groups, alkylsulfonylaminocarbonyl groups, and arylsulfonylaminacarbonyl group. Specific examples include methylsulfonylaminocarbonyl, p-methylphenylsulfonylami-nocarbonyl, acetylaminosulfonyl, and henzoylaminosulfonyl.

The compound represented by general formula (I) will be described in detail below.

The compound represented by general formula (I) comprises in the molecule three or more acid groups selected from sulfonic acid group, carboxylic acid group, and phosphonic acid group. Further, two or more of the acid groups are selected from carboxylic acid group and phosphonic acid group. The substituents X in general formula (I) are acid groups, but neither of X is carboxylic acid group. Acid groups may be in the form of groups forming salts through the dissociation of protons.

In general formula (1), each of R¹ and R² independently represents a substituent. These substituents can be selected from the above-described group of substituents. Each of R¹ and R² preferably represents a halogen atom, alkyl group (including a linear, branched, or cyclic alkyl group), aryl group, heterocyclic group, cyano group, carboxylic acid group, alkoxy group, aryloxy group, heterocyclic oxy group, amino groups (including an anilino group), acylamino group, aminocarbonylamino group, alkoxycarbonylamino group, aryloxycarbonylamino group, sulfamoylamino group, alkyl or arylsulfonylamino group, alkylthio group, arylthio group, heterocyclic thio group, sulfamoyl group, sulfonic acid group, alkyl or arylsulfonyl group, acyl group, alkoxycarbonyl group, carbamoyl group, or imide group. The substituent of more preference is a halogen atom, alkyl group (including a linear, branched, or cyclic alkyl group), aryl group, heterocyclic group, carboxylic acid group, alkoxy group, amino group (including an anilino group), acylamino group, sulfamoyl group, sulfonic acid group, carbamoyl group, or imide group. The substituent of even greater preference is a halogen atom, linear, branched, or cyclic alkyl group having 1 to 6 carbon atoms, aryl group having 6 to 10 carbon atoms, heterocyclic group having 1 to 10 carbon atoms, carboxylic acid group, or sulfonic acid group. The substituent of still greater preference is a bromine atom, iodine atom, methyl group, ethyl group, phenyl group, naphthyl group, thienyl group, furyl group, or pyridyl group.

In general formula (I), m and n represent integers of 0 to 3. When m and n represent 2 or 3, the multiple instances of R¹ and R² may be identical or different. It is preferable for m and n to represent 0 or 1, and preferably for them to represent 0.

In general formula (I), R³ to R⁶ may be identical or different. Each represents a substituted or unsubstituted alkyl group. This alkyl group is identically defined with the alkyl groups at R¹ and R²; when it comprises a substituent, the substituent can be selected from the above-described group of substituents. Each of R⁸ to R⁶ preferably independently represents an alkyl group with 1 to 20 total carbon atoms, more preferably an alkyl group with 1 to 15 total carbon atoms, further preferably an alkyl group with 1 to 10 total carbon atoms, and still further preferably, an alkyl group with 1 to 3 total carbon atoms. Each of R³ to R⁶ preferably independently represents an unsubstituted alkyl group, with a methyl group being preferred.

In general formula (I), R⁷ and R⁸ may be identical or different; each represents a substituted or unsubstituted alkyl group. The alkyl group may be a linear alkyl group (preferably a linear alkyl group having 1 to 30 carbon atoms), a branched alkyl group (preferably a branched alkyl group having 3 to 30 carbon atoms), or a cyclic alkyl group (including an alkyl group having a tricyclic structure, preferably a cycloalkyl group having 3 to 30 carbon atoms or a bicyclic alkyl group having 5 to 30 carbon atoms (a monovalent group obtained by removing a single hydrogen atom from a bicycloalkane having 5 to 30 carbon atoms)). Examples include methyl, ethyl, n-propyl, isopropyl, t-butyl, n-octyl, eicosyl, 2-ethylhexyl, cyclohexyl, cyclopentyl, 4-n-dodecylcyclohexyl, bicyclo[1,2,2]heptane-2-yl, and bicyclo[2,2,2]octane-3-yl.

Of these, a linear alkyl group having 1 to 20 carbon atoms is preferable, a linear alkyl group having 1 to 10 carbon atoms, is more preferred, and a linear alkyl group having 1 to 5 carbon atoms is further more preferred.

The types, number, and positions of substituents on substituted alkyl groups represented by R⁷ and R⁸ are not specifically limited. When R⁷ or R⁸ is a substituted alkyl group, the substituent is preferably a halogen atom, carboxylic acid group, sulfonic acid group, phosphonic acid group, alkylthio group, arylthio group, or heterocyclic thio group; more preferably a carboxylic acid group, sulfonic acid group, or phosphonic acid group; further preferably a carboxylic acid group or phosphonic acid group; moat preferably a phosphonic acid group. A group forming a salt after dissociation of a proton in the acid group is similarly preferable. When the substituted alkyl group is a substituted linear alkyl group, the substitution site is preferably a site located two or more carbon atoms away from the nitrogen atom to which the substituted linear alkyl group substitutes as R⁷ or R⁸, and preferably the terminal carbon atom. The number of substituents is preferably 1.

Each of L¹ to L⁸, which may be identical or different, represents a substituted or unsubstituted methine group. r represents an integer of 0 to 3. When r is 2 or greater, the multiple instances of L² and L³ may be identical or different. When L¹ to L³ represent substituted methine groups, the substituents may be selected from the above-described group of substituents. When any two or more from among L¹ to L³ represent substituted methine groups, the substituents may be bonded together to form a ring.

Specific examples of substituents and specific examples of cases where the substituents bond together to form rings are given below. The examples given below show structures of the polymethine group moiety represented by L¹ to L³ wherein r = 2 and L⁸ is sandwiched between two instances of L² and contains a substituent. An "*" indicates a a position bonding to one of the other two partial structures. 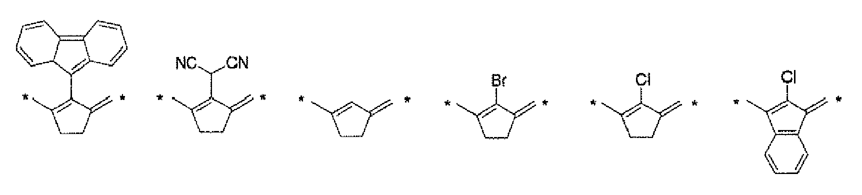 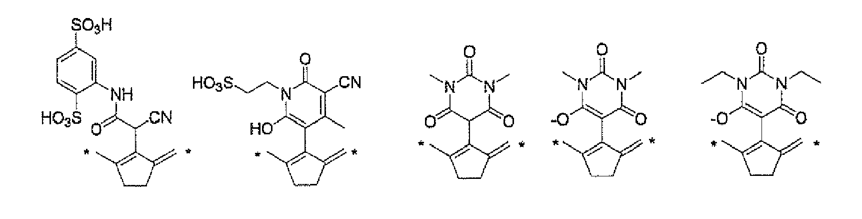 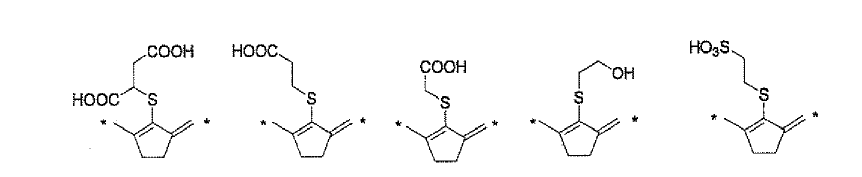 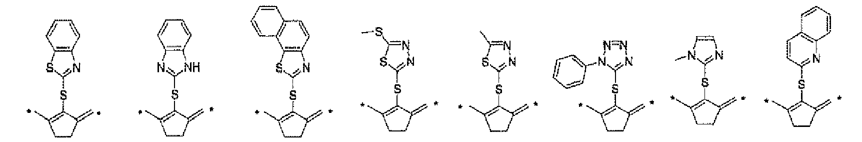 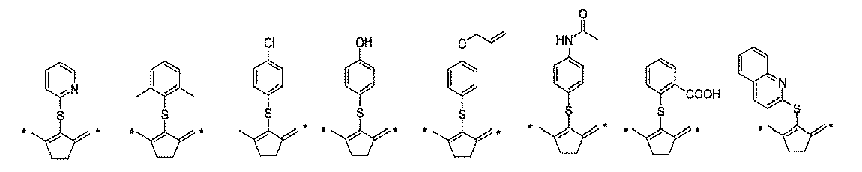 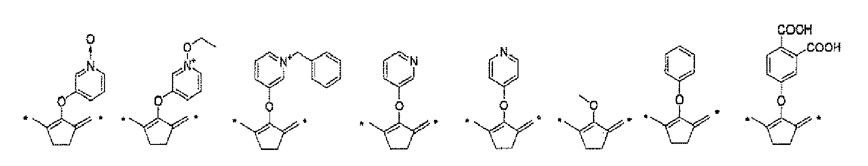 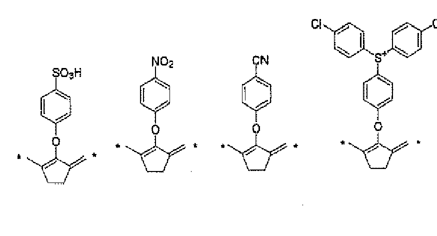 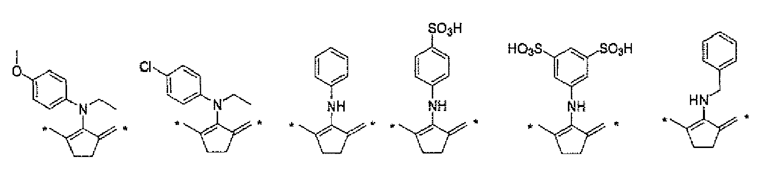 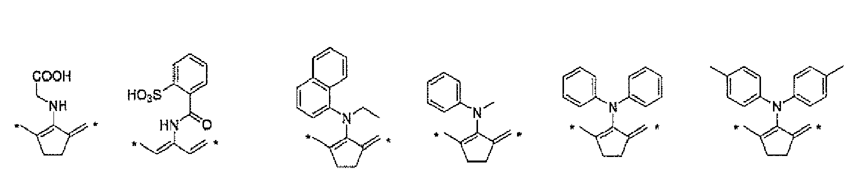 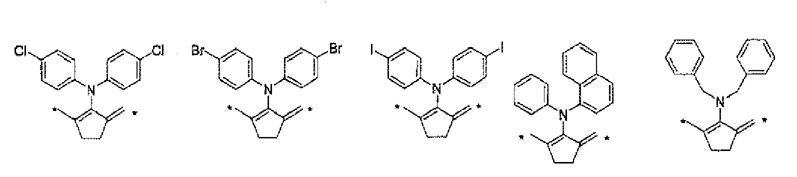 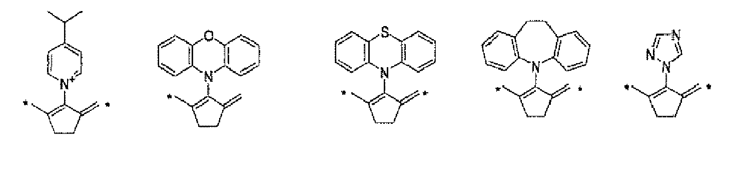 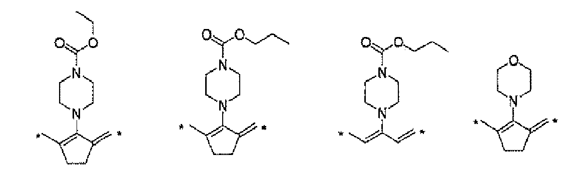 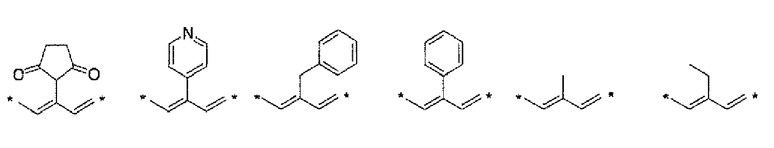 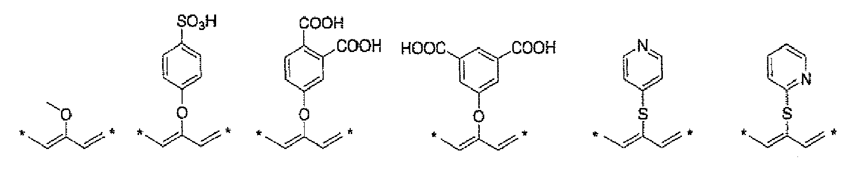 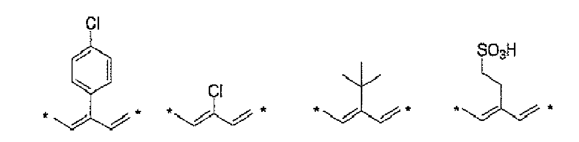 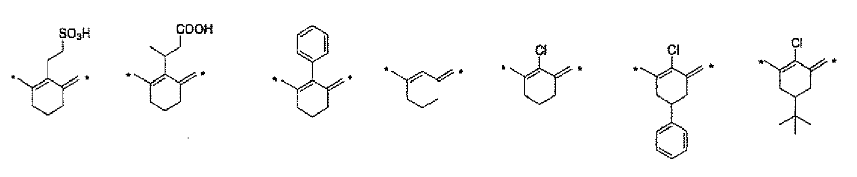 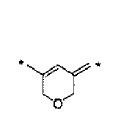 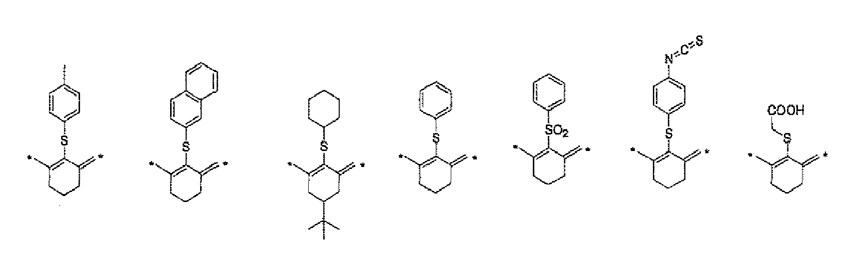 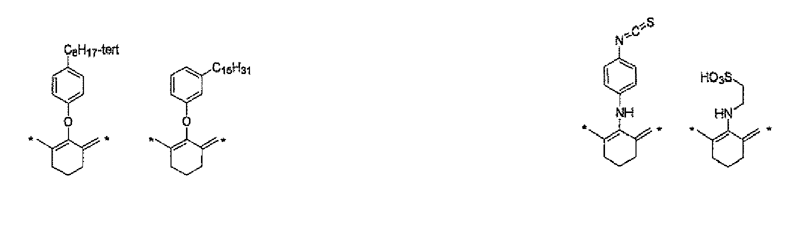 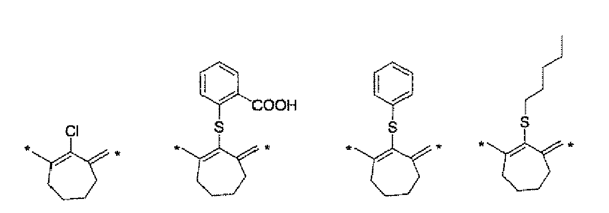 r represents an integer of 0 to 3, preferably 1 to 3, more preferably 2 to 3, and further preferably 3. At least one from among L¹ to L³ preferably represents an unsubstituted methine group, more preferably 1 to 3 from among L¹ to L⁸ represent substituted methine groups and the remainder represent unsubstituted methine groups, further preferably one represents a substituted methine group and the remainder represent unsubstituted methine groups.

When an acid group is in the form of group forming a salt, it preferably forms a salt with a negative charge, or a salt with an alkali metal or alkaline earth metal. Preferable alkali metals and alkaline earth metals are Na and K, with Na being more preferred.

The positive charge of the compound represented by general formula (I) is neutralized by a counter anion either inside or outside the molecule. Examples of such counter anions are: halogen ions such as chlorine ions, bromine ions, and iodine ions; carboxylate ions such as acetate ions, oxalate ions, fumarate ions, and benzoate ions; sulfonate ions such as p-toluene sulfonate, methane sulfonate, butane sulfonate, and benzene sulfonate; sulfuric acid ions; perchloric acid ions; carbonic acid ions; and nitric acid ions. When a group having a negative charge, such as a carboxylate group or sulfonate group, is present within the molecule, it may form an intramolecular salt with a positively charged compound. Preferable examples of extramolecular counter anions include halogen ions, methane sulfonate ions, and sulfuric acid ions. More preferred examples are chloro ions, bromo ions, and methane sulfonate ions.

The compound represented by general formula (I) preferably is the compound represented by general formula (II) above.

When R⁹ in general formula (II) represents an alkyl group, the alkyl group preferably comprises a total of 1 to 20 carbon atoms, more preferably 1 to 15 carbon atoms, and further preferably, 1 to 10 carbon atoms. Specific preferred examples include methyl group, ethyl group, n-propyl group, 2-propyl group, and cyclopropyl group; examples of greater preference include methyl group, ethyl group, n-propyl group, 2-propyl group, and cyclopropyl group.

When R⁹ represents an aryl group, the aryl group preferably comprises a total of 5 to 20 carbon atoms, preferably 5 to 15 carbon atoms, and more preferably, 5 to 10 carbon atoms. Specific preferred examples are a phenyl group, 4-methylphenyl group, 4-phenylphenyl group, and naphthyl group.

When R⁹ represents an arylalkyl group, the arylalkyl group preferably comprises a total of 7 to 30 carbon atoms, more preferably 7 to 20 carbon atoms, and further preferably, 7 to 10 carbon atoms. Specific preferred examples include a benzyl group, phenylethyl group, and phenylpropyl group.

When R⁹ represents a heterocyclic group, the heterocyclic group preferably comprises a total of 1 to 20 carbon atoms, more preferably 1 to 15 carbon atoms, and further preferably, 1 to 10 carbon atoms. Specific preferred examples include a 4-pyridyl group, 2-furyl group, 2-thienyl group, and 2-oxopyrrolidine-1-yl group.

When R⁹ represents an acylamino group, the acylamino group preferably comprises a total of 1 to 20 carbon atoms, more preferably 1 to 15 carbon atoms, and further preferably, 1 to 10 carbon atoms. Specific preferred examples include an acetylamino group, benzoylamino group, pivaloylamino group, and 4-phenylbenzoylamino group.

When R⁹ represents an arylamino group, the arylamino group preferably comprises a total of 1 to 20 carbon atoms, more preferably 1 to 15 carbon atoms, and further preferably, 1 to 10 carbon atoms. Specific preferred examples include an N-phenylamino group, N-tolylamino group, and N,N-diphenylamino group.

When R⁹ represents an N-aryl-N-alkylamino group, the N-aryl-N-alkylamino group preferably comprises a total of 7 to 30 carbon atoms, more preferably 7 to 20 carbon atoms, and more preferably, 7 to 15 carbon atoms. Specific preferred examples include an N-phenyl-N-methylamino group and N-tolyl-N-methylamino group.

When R⁹ represents an arylthio group, the arylthio group preferably comprises a total of 1 to 20 carbon atoms, more preferably 1 to 15 carbon atoms, and further preferably, 1 to 10 carbon atoms. Specific preferred examples include a phenylthio group, tolylthio group, 4-phenylphenylthio group, and naphthylthio group.

When R⁹ represents an aryloxy group, the aryloxy group preferably comprises a total of 1 to 20 carbon atoms, more preferably 1 to 15 carbon atoms, and further preferably, 1 to 10 carbon atoms. Specific preferred examples include a phenyloxy group, 4-acetylaminophenyloxy group, 4-phenylphenyloxy group, and naphthyloxy group.

When R⁹ represents an acylaminoaryloxy group, the acylaminoaryloxy group preferably comprises a total of 7 to 30 carbon atoms, more preferably 7 to 20 carbon atoms, and further preferably, 7 to 15 carbon atoms. A specific preferred example includes a benzoylaminophenyloxy group.

Each of R¹⁰ and R¹¹ independently represents an alkyl group substituted with a carboxylic acid group, sulfonic acid group, or phosphonic acid group. In R¹⁰ and R¹¹, which may be identical or different, it suffices for the alkyl group to be a linear alkyl group (preferably a linear alkyl group having 1 to 30 carbon atoms), branched alkyl group (a branched alkyl group having 3 to 30 carbon atoms), or a cyclic alkyl group (including one having a tricyclic structure, preferably a cycloalkyl group having 3 to 30 carbon atoms, or a bicyclic alkyl group having 5 to 30 carbon atoms (a monovalent group obtained by removing one hydrogen atom from a bicycloalkane having 5 to 30 carbon atoms)). Examples include methyl, ethyl, n-propyl isopropyl, t-brxtyl, n-octyl, eicosyl, 2-ethylhexyl, cyclohexyl, cyclopentyl, 4-n-dodecylcyclohexyl, bicycio[1,2,2]heptane-2-yl, and bicyclo[2,2,2]octane-3-yl. Of these, a linear alkyl group with 1 to 20 carbon atoms is preferable, a linear alkyl group with 1 to 10 carbon atoms is more preferred, and a linear alkyl group with 1 to 5 carbon atoms is further preferred.

In R¹⁰ and R¹¹, neither the number nor position of the carboxylic acid group, sulfonic acid group, or phosphonic acid group is specifically limited. However, each of R¹⁰ and R¹¹ preferably represents an alkyl groups having carboxylic acid group or phosphonic acid group, and more preferably represents an alkyl group having phosphonic acid group. The acid groups may form salts through the dissociation of hydrogen atoms.

When R¹⁰ and R¹¹ are substituted with the above groups, the position of the substituent is preferably two or more carbon atoms away from the nitrogen atom to which each of R¹⁰ and R¹¹ substitutes, preferably the terminal carbon atom.

It is particularly necessary for a fluorescence imaging agent for use within an organism to be water soluble. The water solubility of the near infrared fluorescence imaging agent of the present invention is markedly improved by incorporating three or more acid groups selected from carboxylic acid group, sulfonic acid group, and phosphonic acid group into the above compound. To achieve good water solubility, the total number of sulfonic acid groups, carboxylic acid groups, and phosphonic acid groups is preferably four or more. To facilitate synthesis, the number of sulfonic acid groups, carboxylic acid groups, and phosphonic acid groups is 10 or fewer, preferably 8 or fewer. The improvement in water solubility can be checked by measuring the partition coefficient of the individual compound. For example, the partition coefficient can be checked by measurement in a butanol/water two-phase system. More specifically, the incorporation of three or more sulfonic acid groups yields an n-butanol/water partition coefficient log Po/w of -1.00 or lower.

Water solubility within an organism can be determined based on an index of whether or not the compound settles out or precipitates over time at 36°C when dissolved in physiological saline.

The substance contained as an imaging component in the near infrared fluorescence imaging agent of the present invention is not limited other than that it be the compound represented by general formula (I) or a pharmacologically acceptable salt thereof. Such compounds can be synthesized by the known manufacturing methods for cyanine dye compounds disclosed in "Cyanine Dyes and Related Compounds," F. M. Hamer, John Wiley and Sons, New York, 1964; Cytometry, 10, 3-10 (1989); Cytometry, 11, 418-430 (1990); Cytometry, 12, 723-730 (1990); Bioconjugate Chem. 4, 105-111 (1993), Anal. Biochem., 217, 197-204 (1994); Tetrahedron, 45, 4845-4866 (1989); EP-A-0591820A1; EP-A-0580145A1; and the like. Alternatively, they can be semisynthesized by known methods from commercial cyanine dye compounds. In particular, they can be synthesized by reacting a dianil compound with a heterocyclic compound quaternary salt.

Specific examples of the present invention are given below.

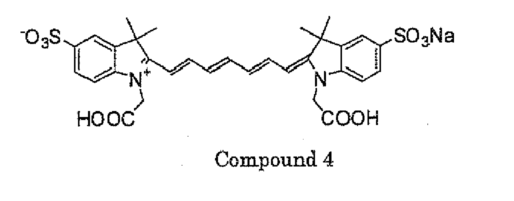

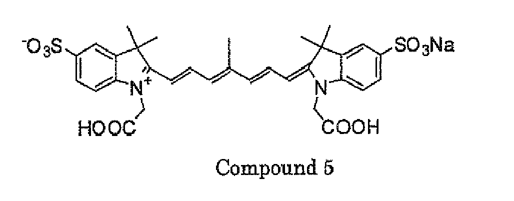

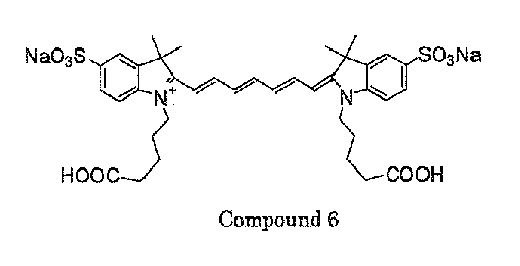

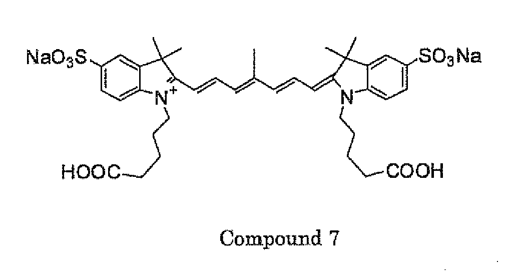

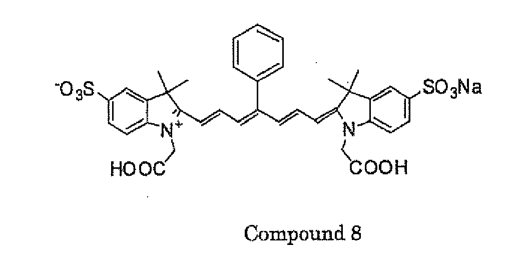

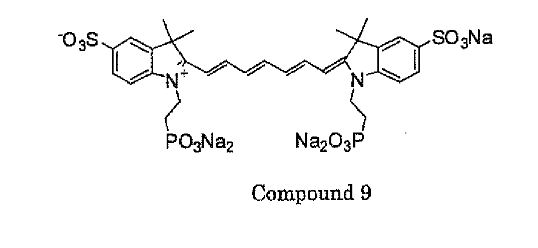

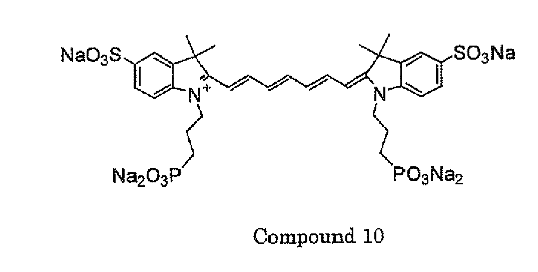

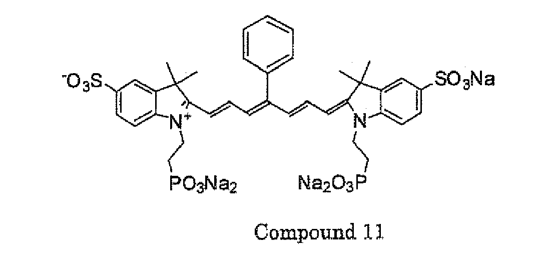

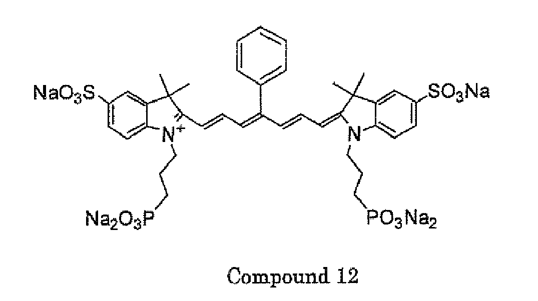

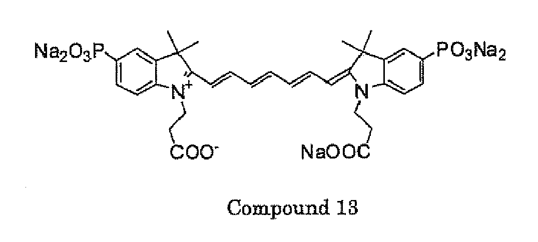

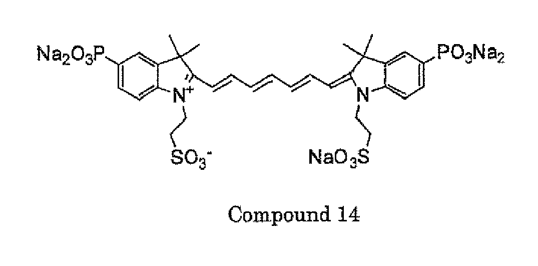

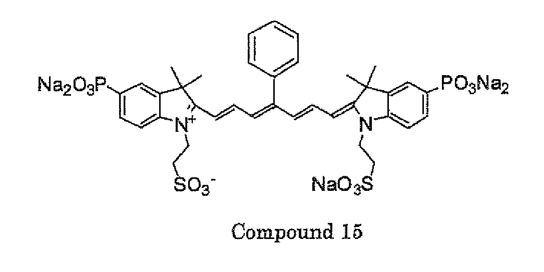

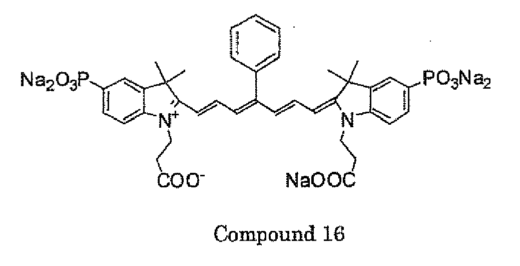

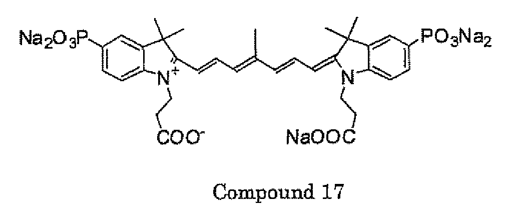

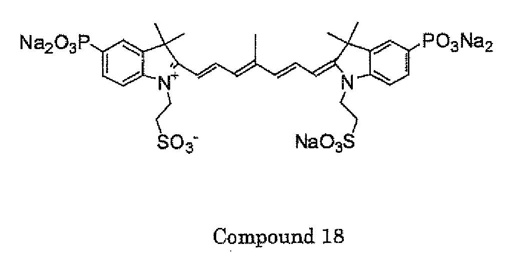

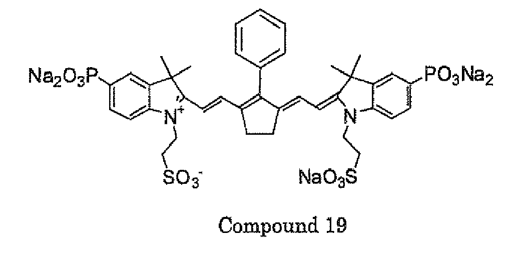

The above compounds, which should be in the near infrared fluorescence imaging agent according to the present invention, exhibit absorption and fluorescence in the near infrared light region of 700 to 1,300 nm, particularly about 700 to 900 nm, and have a molar absorption coefficient of 100,000 or greater.

The near infrared fluorescence imaging agent of the present invention is not specifically limited other than that it comprise the compound represented by general formula (I) and/or a pharmacologically acceptable salt thereof. The compound or salt thereof may be employed singly or in combination in the imaging agent.

Specifically, the imaging agent contains the above compound or a pharmacologically acceptable salt thereof, or the above compound suspended or dissolved in a solvent such as injection-use distilled water, physiological saline, or Ringer's solution. As needed, pharmacologically acceptable additives, such as a support or an excipient, can be added. Examples of these additives include pharmacologically acceptable electrolytes, buffers, substances such as cleansing agents, and substances that enhance stability and solubility by adjusting the osmotic pressure (such as cyclodextrin and ribosomes). Various additives generally employed in related fields can be employed. The near infrared fluorescence imaging agent of the present invention is preferably manufactured for pharmacological use by means of a sterile process.

The imaging agent can be administered to an organism by injection, by spraying, by coating, by intravascular administration (veins, arteries), oral administration, intraperitoneal administration, transdermal administration, subdermal administration, intravesical administration, or intrabronchial administration. The present imaging agent is preferably administered in the form of an aqueous agent, emulsion, or suspension into a blood vessel.

The dose of the near infrared fluorescence imaging agent of the present invention that is employed is not specifically limited other than that the dose employed permit detection of the site to be finally diagnosed, and is suitably adjusted based on the type of compound releasing the near infrared light that is employed, or age, weight, target organ of administration, and the like. Typically, the dose is 0.01 to 100 mg/kg of body weight, preferably 0.01 to 20 mg/kg of body weight, based on the quantity of compound. The imaging agent of the present invention can be suitably employed in various animals in addition to humans. The form, route, and dose administered are suitably determined based on the body weight and conditions of the subject animal.

The compound represented by general formula (I), and, preferably, the compound represented by general formula (II), have a marked tendency to accumulate in tumor tissue. Utilizing this characteristic, it is possible to use the fluorescence imaging agent of the present invention to specifically image tumor tissue. Further, the compound of the present invention has a long half-life in serum, making it possible to anticipate good functioning as a blood vessel imaging agent, as well.

The near infrared fluorescence imaging agent of the present invention can be employed in fluorescence imaging. This method is implemented by a known method. Various parameters such as the excitation wavelength and fluorescence wavelength to be detected are suitably determined to permit optimal imaging and evaluation based on the type of near infrared fluorescence imaging agent being administered and the subject of administration. The time required from when the near infrared fluorescence imaging agent of the present invention is administered to the measurement subject to when measurement by the fluorescence imaging method of the present invention begins varies depending on the type of the near infrared fluorescence imaging agent employed and the subject of administration. For example, when the compound represented by general formula (I) is incorporated into an imaging agent for the purpose of tumor imaging, the time elapsing after administration is considered to be about 4 to 120 hours. In particular, in the case of the compound represented by general formula (II), the time elapsing after administration is considered to be about 24 to 120 hours. When the time elapsed is too short, the fluorescence is excessively strong, making it impossible to clearly distinguish between the target site and other sites. When excessively long, the imaging agent is sometimes discharged from the body.

Research by the present inventors based on SF-64 revealed for the first time that cyanine compounds form complexes with albumin in plasma, thereby accumulating in tumors. The compound represented by general formula (I) having two or more of acid groups selected from carboxylic acid group and phosphonic acid group has a higher ability to bind albumin than SF-64.

Due to the high ability to bind albumin of the compound of the present invention, it can bind to albumin-binding proteins such as SPARC, cubilin, and TGF-*beta,* Thus, the compound of the present invention can be employed as an imaging agent in the course of imaging disease in which albumin-binding proteins play primary roles and are expressed in greater amounts than in normal tissue. The albumin-binding protein is preferably selected from among SPAR, cubilin, and TGF-*beta,* and is preferably SPAR.

The compound of the present invention or a pharmacologically acceptable salt thereof thus functions as a detecting agent for SPARC-expressing tissue and can be employed as a diagnostic drug for all pathology relating to SPARC. The pathology for which the compound of the present invention can be employed as an imaging agent includes abnormal reproductive states, tissue reconstruction, overformation, and excessive wound healing in any physical tissue including soft tissue, connective tissue, bone, solid organs, and blood vessels. Specific examples include cancer, diabetes and related retinal disorders, inflammation, arthritis, restenosis in blood vessel, sections of artificial vascular transplants, and intravascular devices.

The types of tumors that are detected by the compound of the present invention are those that are generally observed in mammals, including humans. These tumors can be generated by inoculation of test animals and the like. The tumors are also known as "neoplasms," and come in many types and forms. The tumors to which the compound of the present invention is applied as an imaging agent are not limited, and may be any specific type or form of tumor. The compound of the present invention is useful for imaging tumor cells, related interstitial cells, solid tumors, and tumors relating to soft tissue, such as human soft tissue sarcomas. The tumor or cancer can be located in the mouth cavity, pharynx, digestive organs, respiratory organs, bones, joints (such as bone metastasis), soft tissue, skin (such as melanoma), breasts, genitalia, urinary system, eyes, eyeball sockets, brain, central nervous system (such as gliomas), or the endocrine system (such as the thyroid gland), and are not limited to primary tumors or cancers. Examples of tissues relating to the mouth cavity include tongue and mouth tissues. Cancers can occur in digestive system tissue, including the esophagus, stomach, duodenum, small intestine, colon, rectum, anus, liver, gall bladder, and pancreas. Respiratory cancers can occur in the pharynx, lungs, and bronchia. Tumors can occur in the cervix, uterus, ovaries, vulva, vagina, prostate, testicles, and penis, constituting the male and female genitalia, as well as in the bladder, kidneys, renal pelvis, and urethra, constituting the urinary system. Tumors and cancers can occur in the head and/or cervix. Tumors and cancers can also occur within the lymph tissue system. Examples thereof include Hodgkin's disease and non-Hodgkin's lymphomas. Tumors and cancers can occur in the hematogenic system. Examples thereof include multiple myeloma and leukemia, such as acute lympholeukemia, chronic lymphocytic leukemia, acute myelitic leukemia, and chronic myelitic leukemia. The tumor is preferably positioned in the bladder, liver, pancreas, ovaries, kidneys, esophagus, stomach, intestines, brain, skin, or breasts.

Albumin has affinity for SPARC. Accordingly, the compound of the present invention accumulates at lesion area expressing SPARC while accumulating little or not at all in healthy tissue.

The expression of SPARC protein in samples can be detected and quantified by any suitable method that is known in the field. Examples of suitable methods for detecting and quantifying proteins are the western blot method, enzyme-linked immunosorbent assay (ELISA), silver dye method, BCA assay (described in Smith et al., Anal. Biochem., 150, 76-85 (1985)), the Lowry protein assay, which is a form of protein-copper complex-based colorimetric analysis (described, for example, in Lowry et al., J. Biol. Chem., 193, 265-275 (1951)), and Bradford protein analysis based on the absorbance change of Coomassie blue G-250 in the course of protein binding (described, for example, in Bradford et al., Anal. Biochem., 72, 248 1976)). Tumor biopsies can be conducted by any conventional method, or conducted immunohistologically using anti-SPARC antibody (either monoclonal or polyclonal) in combination with a suitable visualization system (that is, HRP substrate and HRP-coupled secondary antibodies).

### Examples

The present invention will be described more specifically below through examples. However, the scope of the present invention is not limited to the examples set forth below. The compound numbers given in the examples below correspond to the above-described compound examples. The names of the compounds indicated in the embodiments below do not necessarily conform to IUPAC naming conventions.

In the examples below, the serial numbers of the compounds and the compound structures correspond to the numbers of the compounds given by way of example above.

Intermediates for synthesizing the compound of the present invention were obtained by the following procedure.

5-Sulfo-2,3,3-trimethylindoleine was prepared by a known synthesis method (Japanese Unexamined Patent Publication (KOKAI) Heisei No. 2-233658).

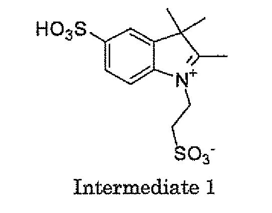

Intermediate 1 (a heterocyclic quaternary salt compound) was prepared by a method described in the patent literature (Published Japanese Translation (TOKUHYO) No. 2002-526458 of a PCT International Publication (WO00/16810)).

That is, 2-cbloroethanesulfononyl chloride was added dropwise over two hours while stirring at room temperature to 5-sulfo-2,3,3-trimethylincioleine dissolved in DMAC, then the mixture was stirred for another three hours. Methanol was added dropwise over 30 minutes, then an aqueous sodium hydroxide solution was added dropwise over one hour and stirred for another hour at room temperature. When the reaction was completed, acetonitrile was added dropwise over two hours and the mixture was left standing overnight at room temperature to allow crystals to precipitate. The crystals were collected by filtration, washed with a DMAc/methanol/water/acetonitrile = 5/4/4/40 solution followed by acetonitrile, and dried under reduced pressure to obtain intermediate 1. 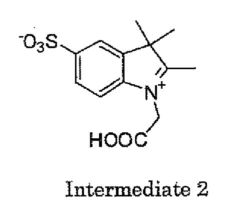 5-Sulfo-2,3,3-trimethylindoleine(1 equivalent), bromo acetic acid (1.5 equivalents), and triethylamine (1 equivalent) were stirred in o-dichlorobenzene at 120°C for 6 hours. After completion of the reaction, the supernatant was removed by decantation. The residue was washed with ethyl acetate. The obtained crude product was recrystallized from water/methanol/ethyl acetate to obtain Intermediate 2 as pale read white solid.

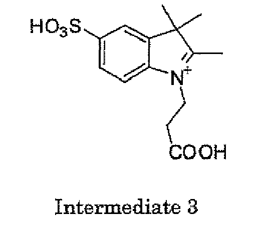

Intermediate 3 was obtained in a similar manner to that of intermediate 2 by using 5-sulfo-2,3,3-trimethylindoleine and2-bromopropionic acid.

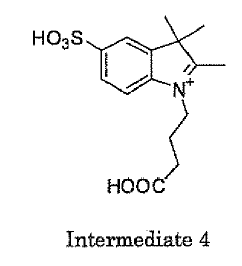

Intermediate 4 was obtained in a similar manner to that of intermediate 2 by using 5-sulfo-2,3,3-trimethylindoleine and 4-bromo-n-butyric acid. 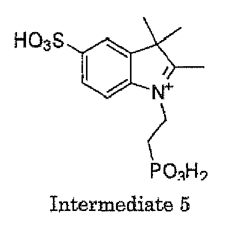 5-Sulfo-2,3,3-trimethylindoleine (1 equivalent), diethyl 2-bromoethylphosphate (1.1 equivalents), and triethylamine(1.5 equivalents) were stirred in toluene at 135°C for 6 hours. After completion of the reaction, the solvent was evaporated under reduced pressure. The crude product was added with 6N hydrochloric acid, and refluxed for 24 hours. After completion of the reaction, the solvent was evaporated under reduced pressure to obtain crude product containing intermediate 5 as red oil. 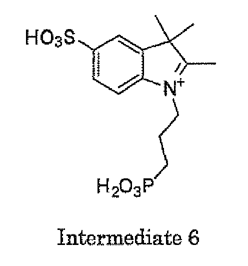 5-Sulfo-2,3,3-trimethylindoleine (1 equivalent), diethyl 2-bromopropylphosphate (1.1 equivalents), and triethylamine(1.5 equivalents) were stirred in toluene at 135°C for 6 hours. After completion of the reaction, the solvent was evaporated under reduced pressure. The crude product was added with 6N hydrochloric acid, and refluxed for 24 hours. After completion of the reaction, the solvent was evaporated under reduced pressure to obtain crude product containing intermediate 6 as red oil. 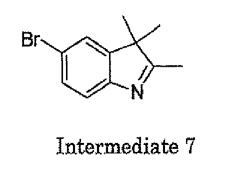 Intermediate 7 was prepared in accordance with the method described in Bioconjugate Chem. 18, 2178-2190 (2007).

Specifically, 4-bromophenyl hydrazine monohydrochloride and 3-methyl-2-butanone are dissolved in ethanol. The obtained solution was added with concentrated sulfuric acid dropwise and reflexed under stirring for 6 hours. After completion of the reaction, the solvent was concentrated under reduced pressure. The resultant was added with aqueous sodium hydroxide to achieve pH 8 and the oil layer was extracted with ethyl acetate. The oil layer was washed with saturated brine, dried over sodium sulfate, and purified by silica gel column chromatography (hexane: ethyl acetate=3:1) to obtain intermediate 7 as brown liquid. MS(ESI+) 238 [M+1]+

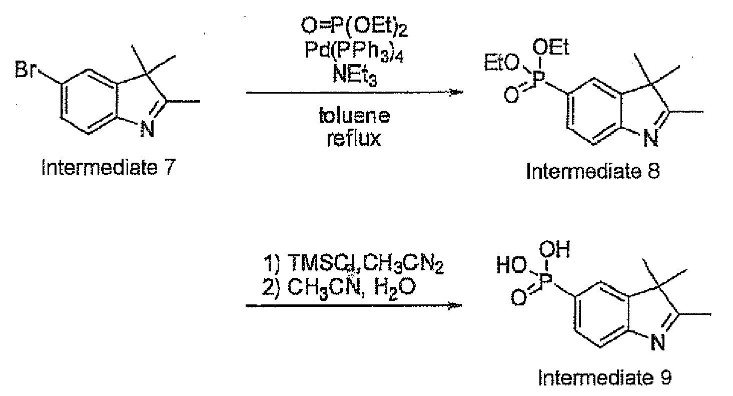

Intermediate 7 (1 equivalent), diethylphosphonate (1.1 equivalents), triethylamine (1.1 equivalents), and tetrakis(triphenylphosphirle)palladium (0.06 equivalents) were added to toluene. The obtained solution was stirred at 95°C under nitrogen atmosphere for 6 hours. After completion of the reaction, excess ethyl acetate was added to the solution, and the generated precipitate was removed by filtration, and the solvent was evaporated. The obtained intermediate 8 was dissolved in acetonitrile. The solution was added with trimethylsilyl bromide (6 equivalents) and stirred at room temperature for 5 hours. The excess trimethylsilyl bromide and the solvent were evaporated under reduced pressure. The residue was added with acetonitrile:water=2:1 and stirred at room temperature for 12 hours. The solvent was evaporated under reduced pressure. The obtained crude product was added with methanol. The generated insoluble matter was removed by filtration. The filtrate was added with ethyl acetate. The generated oil was collected by separation treatment, and the oil was dried under reduced pressure to obtain intermediate 9.

Intermediate 9: 1H NMR (300MHz, MeOD) δ1.50 (s, 6H), 3.34 (s, 3H), 7.72-7.98 (m, 1H), 7.85-7.99 (m, 2H), MS(ESI+) 240 [M+1]+

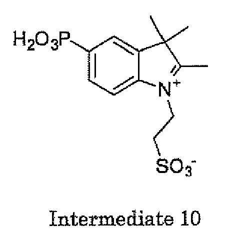

Intermediate 9 was added with DMAc and triethylamine (1 equivalent). The solution was added with 2-chloroethane sulfonyl chloride (3 equivalents) dropwise, while being kept at room temperature. The solution was stirred for 4 hours, and added with aqueous 1N sodium hydroxide. The solution was stirred at room temperature for one hour and added with large excess acetone dropwise. The obtained oil was collected by separation treatment. The oil was dissolved in methanol again. The solution was added to acetone dropwise and the precipitated powder was collected by filtration to obtain intermediate 10. MS(ESI+) 348 [M+1]+ 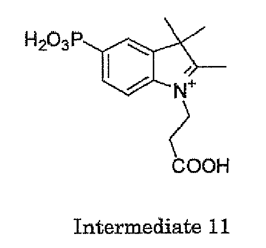 Intermediate 11 was obtained in a similar manner to that of intermediate 2 by using 2-bromopropionic acid, and intermediate 10 that was obtained in a similar manner to that of intermediate 9. MS(ESI+) 312 [M+1]+ 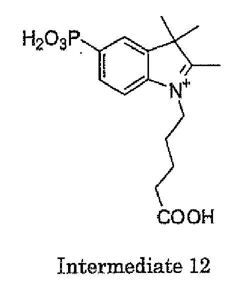 Intermediate 12 was obtained in a similar manner to that of intermediate 2 by using 4-bromo-n-butyric acid, and intermediate 10 that was obtained in a similar manner to that of intermediate 9. 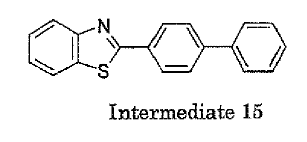 3-Phenylpyridine (1 equivalent) and 2 chlorobenzothiazole (2 equivalents) were stirred in DMAc at 100°C for three hours. After completion of the reaction, acetone was added. The solid was collected by filtration, yielding intermediate 15.

The dianil compounds indicated below were obtained or synthesized by the method described in the patent literature (Published Japanese Translation (TOKUHYO) No. 2008-526802 af a PCT International Publication (WO2006/072580)).

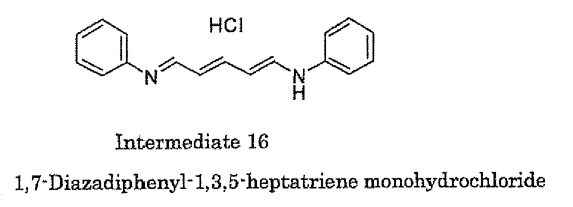

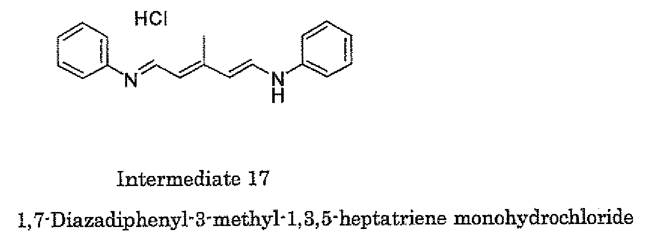

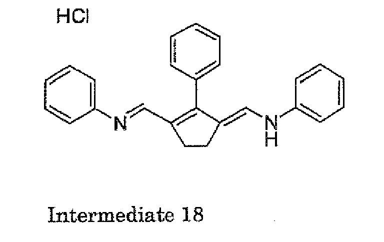

The compounds and salts thereof indicated below were manufactured by the methods indicated below, for example, using the above heteroquaternary salt compounds and dianil compounds or benzthiazole compounds.

A known heteroquaternary compound (5 moles), or one synthesized by a known method or the above-described synthesis method, and a known dianil compound (10 mmol), or one synthesized by a known method or the above-described synthesis method, or a benzthiazole compound (10 mmoles), is stirred for four hours at room temperature in a methanol solution in the presence of triethylamine (25 mmol) and acetic anhydride (45 mmol). To the reaction mixture is added sodium acetate (33 mmol) and the mixture is stirred for 30 minutes at room temperature. The crystals generated are removed by filtration and washed with methanol. When the presence of products other than the target compound is observed by reverse-phase thin layer chromatography, purification is conducted by size exclusion chromatography with methanol as the developing solvent or by reverse phase chromatography with a water/methanol mixed solvent as the developing solvent.

The condition for the HPLC analysis is as follows.

HPLC Column: TOSOH TSKgel ODS-100V, 150 X 4.6mm

Column temperature: 40 °C

Flow rate 1.0 mL/min

eluent: A) water/acetic acid/triethylamine (100/0.1/0.1), B) acetonitrilelacetic acid/triethylamine (100/0.110.1)

gradient: 0-100%B(0-30 min)

Detection: Absorbance at 254 nm and 780 nm

Compound 4: Prepared by the above synthesis method using intermediates 2 and 16.

1H NMR (400MHz, D₂O) δ 1.68 (s, 12H), 4.60 (s, 4H), 6.11 (d, 2H), 6.54 (dd, 2H), 7.33 (d, 2H), 7.49 (dd, 1H), 7.74-7.92 (m,6H), HPLC 10.7min, λ max=744nm Compound 5: Prepared by the above synthesis method using intermediates 2 and 17.

1H NMR (400MHz, D₂O) δ 1.68 (s, 12H), 2.30 (s, 3H), 4.60 (s, 4H), 6.25 (d, 2H), 6.54 (dd, 2H), 7.25 (d, 2H), 7.74-7.92 (m,6H) HPLC 12.2min, λmax=759nm Compound 6: Prepared by the above synthesis method using intermediates 4 and 16.

1H NMR (400MHz, D₂O) δ 1.60 (s, 12H), 1.90-2.05 (m, 8H), 2.90 (s, 4H), 4.60 (s, 4H), 6.11 (d, 2H), 6.54 (dd, 2H), 7.33 (d, 2H), 7.49 (dd, 1H), 7.74-7.92 (m,6H), λ max=759nm Compound 7: Prepared by the above synthesis method using intermediates 4 and 17.

1H NMR (400MHz, D₂O) δ 1.65 (s, 12H), 1.90-2.20 (m, 8H), 2.90 (s, 4H), 4.60 (s, 4H), 6.11 (d, 2H), 6.54 (dd, 2H), 7.33 (d, 2H), 7.49 (dd, 1H), 7.74-7.92 (m,6H), λ max=752nm Compound 8: Prepared by the above synthesis method using intermediates 2 and 15.

1H NMR (400MHz, D₂O) δ 1.21 (s, 12H), 4.58 (s, 4H), 6.15 (d, 2H), 6.68 (d, 2H), 6.85-6.95 (m, 2H), 7.10 (d, 2H), 7.28 (dd, 2H), 7.46-7.56 (m, 3H), 7.65-7.72 (m, 4H), MS(ESI+) 733 [M+1]+, HPLC 12.4min, λmax=785nm

Compound 9: Prepared by the above synthesis method using intermediates 5 and 16.

1H NMR (400MHz, D₂O) δ 1.60 (s, 12H), 2.07 (m, 4H), 4.28-4.33 (m, 4H), 6.27 (d, 2H), 6.57 (dd, 2H), 6.33 (d, 2H), 7.45 (dd, 1H), 7.74-7.88 (m,6H), MS(ESI+) 758 [M+1]+, HPLC 10.3min, λmax=773nm

Compound 10: Prepared by the above synthesis method using intermediates 6 and 16.

1H NMR (400MHz, D₂O) δ 1.23 (s, 12H), 1.7-1.9 (m, 4H), 3.2-3.4 (m, 4H), 4.0-4.2 (m, 4H), 6.27 (d, 2H), 6.57 (dd, 2H), 6.33 (d, 2H), 7.45 (dd, 1H), 7.74-7.88 (m,6H), HPLC 10.5min, λ max=775nm

Compound 11: Prepared by the above synthesis method using intermediates 5 and 15.

1H NMR (400MHz, D₂O) δ 1.07 (s, 12H), 1.92-2.10 (m, 4H), 4.15-4.28 (m, 4H), 6.40 (d, 2H), 6.69 (dd, 2H), 7.19 (dd, 2H), 7.45-7.57 (m, 5H), 7.58-7.69 (m, 6H), HPLC 13.6min, λ max=786nm

Compound 12: Prepared by the above synthesis method using intermediates 6 and 15.

1H NMR (400MHz, D₂O) δ 1.23 (s, 12H), 1.7-1.9 (m, 4H), 3.2-3.4 (m, 4H), 4.0-4.2 (m, 4H), 6.26-6.44 (m, 4H), 6.74-6.84 (m, 2H), 7.15-7.71 (m, 13H), HPLC 12.8min, λ max=783nm Compound 13: Prepared by the above synthesis method using intermediates 11 and 16. 1H NMR (400MHz, D₂O) δ 1.59 (s, 12H), 2.61 (t, 4H), 4.20-4.40 (m, 4H), 6.26 (d, 2H), 6.53 (dd, 2H), 7.254 (d, 2H), 7.45 (dd, 1H), 7.55-7.95 (m, 6H), λ max=781nm Compound 14: Prepared by the above synthesis method using intermediates 10 and 16. 1H NMR (300MHz, D₂O) δ 1.64 (s, 12H), 3.35 (t, 4H), 4.43 (t, 4H), 6.30 (d, 2H), 6.53-6.66 (m, 2H), 7.26-7.33 (m, 2H), 7.45-7.59 (m, 1H), 7.64-7.79 (m, 4H), 7.86-7.99 (m, 2H), HPLC 11.0min, λ max=776nm

Compound 15: Prepared by the above synthesis method using intermediates 10 and 15. 1H NMR (400MHz, D₂O) δ 1.33 (s, 12H), 3.15-3.22 (m, 4H), 4.38-4.45 (m, 4H), 6.48 (dd, 2H), 6.83 (d, 2H), 7.30-7.39 (m, 4H), 7.47 (dd, 2H), 7.61-7.69 (m, 3H), 7.73 (dd, 2H), 7.78-7.86 (m, 4H), HPLC 10.8min, λ max=788nm

Compound 16: Prepared by the above synthesis method using intermediates 11 and 15.

1H NMR (400MHz, D₂O) δ 1.32 (s, 12H), 2.65-2.73 (m, 4H), 4.29-4.41 (m, 4H), 6.44 (dd, 2H), 6.79 (d, 2H), 7.26-7.37 (m, 4H), 7.47 (dd, 2H), 7.61-7.69 (m, 3H), 7.70-7.85 (m, 4H), λ max=792nm

Compound 17: Prepared by the above synthesis method using intermediates 11 and 17. 1H NMR (400MHz, D₂O) δ 1.75 (s, 12H), 2.38 (s, 3H), 3.65 (t, 4H), 4.34 (t, 4H), 6.43 (dd, 2H), 6.64 (d, 2H), 7.33 (dd, 2H), 7.84 (dd, 4H), 8.08 (dd, 2H), λ max=791nm Compound 18: Prepared by the above synthesis method using intermediates 10 and 17. 1H NMR (400MHz, D₂O) δ 1.72 (s, 12H), 2.40 (s, 3H), 3.23 (t, 4H), 4.46 (t, 4H), 6.43 (dd, 2H), 6.68 (d, 2H), 7.31 (dd, 2H), 7.87 (dd, 4H), 8.10 (dd, 2H), λ max=789nm Compound 19: Prepared by the above synthesis method using intermediates 11 and 18. 1H NMR (400MHz, D₂O) δ 1.32 (s, 12H), 2.65-2.73 (m, 4H), 4.29-4.41 (m, 4H), 6.44 (dd, 2H), 6.79 (d, 2H), 7.26-7.37 (m, 4H), 7.47 (dd, 2H), 7.61-7.69 (m, 3H), 7.70-7.85 (m, 4H), λ max=813nm

Compound A as below was prepared from intermediates 1 and 16 in accordance with the above method and used as a comparative compound.

Compound B (SF-64) was prepared from intermediates 1 and 17 as described in paragraph [0058] of Japanese Patent 3,507,060 and used as a comparative compound.

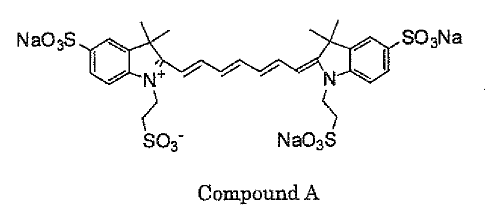

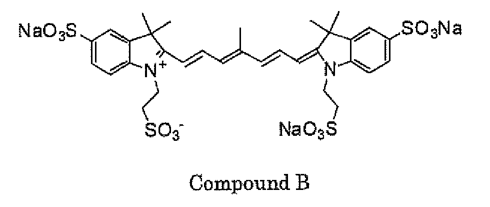

### Test Example 1

Serum protein binding rate test by ultracentrifugation

The test substance was dissolved to 5 µg/mL in calf serum, vibrated for 20 minutes at 37°C, and subjected to density gradient centrifugation (1,000,000 x g, 24 h) using NaBr. Following the centrifugation, the dye concentrations in the albumin fraction, lipoprotein fraction, and the remaining fractions were quantified by HPLC. The results are given in Table 1.

**Table 1**

| Compound | Level | Protein binding rate (%) | | |
|---|---|---|---|---|
| | | Albumin | lipoprotein | other |
| Compound B | Comparative Example | 60 | 1 | 39 |
| ICG | Comparative Example | 18 | 81 | 1 |
| Compound 4 | Invention | 76 | 2 | 21 |
| Compound 5 | Invention | 83 | 2 | 15 |
| Compound 6 | Invention | 72 | 2 | 26 |
| Compound 7 | Invention | 75 | 2 | 23 |
| Compound 8 | Invention | 86 | 2 | 12 |
| Compound 9 | Invention | 77 | 2 | 21 |

From Table 1, it is clear that the compounds of the present invention having the structure shown in general formula (I) exhibit high binding rates for albumin in particular among the protein components present in serum. It is also clear that compounds with poor water solubility, such as ICG, bonded to lipoprotein among the protein components in serum.

### Test Example 2

Plasma protein binding rate test by equilibrium dialysis method

Plasma protein binding rates of the compounds were determined by equilibrium dialysis method (W. Lindner et al, J.Chromatography, 1996, 677, 1-28). Each test compound was dissolved in distilled water at concentration of 200 µM. The solution was added to the plasma of mouse, dog, rabbit, monkey and human respectively, at the amount to obtain the final concentration of 5 µ M. This solution(300 µ L) and PBS solution (500 µ L ) were added to each dialysis cell of equilibrium dialysis unit RED device (Pierce Biotechnology), and the cells were shaken at 100 rpm at 37°C. After 4 hours, samples of plasma and the buffer were collected, and the fluorescence intensity of each was measured by EnVision(GE Corporation). After the concentrations were calculated from a calibration curve, the concentrations (%) of free test compound in plasma were determined. The results are shown in Tables 2 and 3.

**Table 2**

| | Compound | Plasma protein binding rate (%) | | | | |
|---|---|---|---|---|---|---|
| | | mouse | rabbit | dog | monkey | human |
| Invention | Compound 4 | 57 | 67 | 50 | 71 | 74 |
| Invention | Compound 6 | 65 | 88 | 59 | 79 | 88 |
| Invention | Compound 9 | 46 | 61 | 50 | 67 | 56 |
| Invention | Compound 10 | 53 | 65 | 52 | 65 | 62 |
| Invention | Compound 11 | 91 | 98 | 83 | 97 | 98 |
| Invention | Compound 12 | 91 | 95 | 82 | 93 | 95 |
| Invention | Compound 13 | 60 | 87 | 70 | 90 | 89 |
| Invention | Compound 14 | 65 | 87 | 71 | 95 | 94 |
| Invention | Compound 15 | 70 | 94 | 74 | 96 | 93 |
| Invention | Compound 16 | 77 | 95 | 77 | 92 | 95 |
| Invention | Compound 17 | 63 | 79 | 59 | 91 | 93 |
| Invention | Compound 18 | 59 | 82 | 65 | 95 | 96 |
| Invention | Compound 19 | 90 | 93 | 88 | 98 | 98 |
| Comparative Example | Compound B | 60 | 88 | 61 | 93 | 92 |
| Comparative Example | Compound A | 27 | 79 | 29 | 87 | 82 |

**Table 3**

| | Compound | Standard deviation of the binding rate among animal species ( σ ) |
|---|---|---|
| Invention | Compound 4 | 9.0 |
| Invention | Compound 6 | 12 |
| Invention | Compound 9 | 7.5 |
| Invention | Compound 10 | 5.7 |
| Invention | Compound 11 | 4.2 |
| Invention | Compound 12 | 4.8 |
| Invention | Compound 13 | 12 |
| Invention | Compound 14 | 12 |
| Invention | Compound 15 | 11 |
| Invention | Compound 16 | 8.4 |
| Invention | Compound 17 | 14 |
| Invention | Compound 18 | 15 |
| Invention | Compound 19 | 4.1 |
| Comparative Example | Compound B | 15 |
| Comparative Example | Compound A | 27 |

Tables 2 and 3 show that the compounds of the present invention represented by the general fomula (I) exhibit smaller difference in the plasma protein binding rate among animal species compared to that of SF-64. It will be understood that difference in the plasma protein binding rate among animal species tends to get smaller by replacing sulfonic acid group at X with phosphonic acid group from a comparison of the standard deviation of compound A and compound 15 in Table 3, and by replacing sulfonic acid group at the acid group substituted in R7 group and R8 group in the compound represented by the general fomula (I) with carboxylic acid group, and further with phosphonic acid group from a comparison of the standard deviation of compound A, 4, 6, 9, and 10 in Table 3.

### Effect of the Invention

The present invention provides a novel cyanine compound. The cyanine compound tends to accumulate in diseased parts, such as tumors, and is suitable as an imaging component in a near infrared fluorescence imaging agent. The above cyanine compound exhibit little difference of tumor outlining ability among the animal species, and thus the result that will be obtained when the test compound is administered to human can be easily predicted from the result of the animal test, The near infrared fluorescence imaging agent containing the compound of the present invention is characterized by outlining tumors for extended periods.

## Claims

1. A compound represented by general formula (I) below, or a pharmacologically acceptable salt thereof: 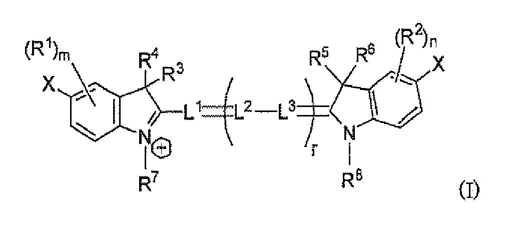 wherein X represents sulfonic acid group or phosphoric acid group; each of R¹ and R² independently represents a substituent; R³ to R⁶ may be identical or different and each represents an optionally substituted alkyl group; each of R⁷ and R⁸ independently represents an optionally substituted alkyl group; L¹ to L⁸ may be identical or different and each represents an optionally substituted methine group, r represents an integer of from 0 to 3, and when r is 2 or more, the plural L² and the plural L³ may be the same or different, respectively; each of m and n independently represents an integer of 0 to 3; at least one of the substituents is an acid group selected from sulfonic acid group, carboxylic acid group, and phosphonic acid group or a group comprising the acid group; and two or more of the acid groups and X are selected from carboxylic acid group and phosphonic acid group.

2. The compound according to claim 1 represented by general formula (II) below, or a pharmacologically acceptable salt thereof 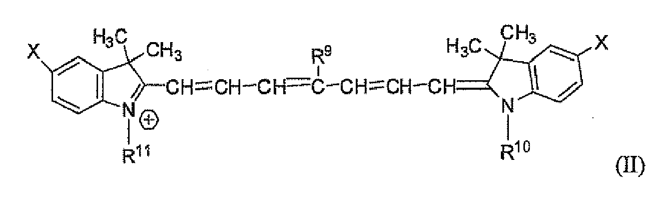 wherein each of R¹⁰ and R¹¹, which may be identical or different, represents an alkyl group substituted with carboxylic acid group, sulfonic acid group, or phosphonic acid group and R⁹ represents hydrogen atom, an alkyl group, an aryl group, an arylalkyl group, a heterocyclic group, an acylamino group, an arylamino group, an N-aryl-N-alkylamino group, an arylthio group, an aryloxy group, or an acylaminoaryloxy group.

3. The compound according to claim 2, or a pharmacologically acceptable salt thereof, wherein R⁹ represents hydrogen atom, methyl group, or phenyl group.

4. The compound according to claim 2 or 3, or a pharmacologically acceptable salt thereof, wherein each of R¹⁰ and R¹¹ represents an alkyl group substituted with sulfonic acid group or phosphonic acid group.

5. The compound according to claim 2 or 3, or a pharmacologically acceptable salt thereof, wherein each of R¹⁰ and R¹¹ represents an alkyl group substituted with phosphonic acid group.

6. The compound according to any one of claims 1 to 5, or a pharmacologically acceptable salt thereof, wherein X represents sulfonic acid group.

7. The compound according to any one of claims 1 to 6, or a pharmacologically acceptable salt thereof, wherein one or more of carboxylic acid group, sulfonic acid group, and phosphonic acid group form salts with sodium ions.

8. A near infrared fluorescence imaging agent comprising the compound, or pharmacologically acceptable salt thereof, according to any one of claims 1 to 7.

9. The near infrared fluorescence imaging agent according to claim 8, which is used to image a tumor.

10. The near infrared fluorescence imaging agent according to claim 9, which is used to image a tumor of a type expressing a high level of SPARC.

11. The near infrared fluorescence imaging agent according to claim 9 or 10, which is used to image tumors occurring in the mouth cavity, pharynx, digestive organs, respiratory organs, bones, joints, soft tissue, skin, breasts, genitalia, urinary system, eyes, eyeball sockets, brain, central nervous system, or endocrine system.
